# EUROPEAN PATENT APPLICATION

(11) **EP 0 880 968 A1**
(43) Date of publication of application: **02.12.1998**
(21) Application number: 97108593.1
(22) Date of filing: 28.05.1997
(51) Int. Cl.: A61K 38/09, A61K 47/22, A61K 47/12, A61K 47/18

(54) **Pharmaceutical compositions of peptides having low solubility in physiological medium**

(71) Applicant: Applied Research Systems ARS Holding N.V., Curaçao (AN)
(72) Inventor: Richardson, Peter, 00040 Rome (IT)
(74) Representative: Vannini, Mario

(57) **Abstract**

Pharmaceutical compositions are described, which comprise:
a) a peptide poorly soluble in aqueous physiological saline solution, as active ingredient;
b) a non-ionic aromatic hydrotropic pharmaceutically acceptable agent; and
c) a physiological aqueous solution.

## Description

### FIELD OF THE INVENTION

The present invention relates to a pharmaceutical composition comprising:
a) a peptide poorly soluble in aqueous physiological saline solution, as active ingredient;
b) a non-ionic aromatic hydrotropic pharmaceutically acceptable agent; and
c) a physiological aqueous solution.

According to a preferred embodiment of the invention, the peptide (a) is an LHRH analogue.

### BACKGROUND OF THE INVENTION

It is often necessary to improve the solubility of a drug in physiological media in order to achieve effective clinical performance of injectable formulations of the drug. Peptide drugs are often poorly soluble in physiological media due to the presence of hydrophobic substituents.

Solubility problems can also lead to poor absorption by other routes of administration and in some cases suitable solubilising agents can aid the absorption of the drug by other routes, for example oral or nasal.

Luteinising hormone releasing hormone (LHRH or GnRH) is a decapeptide secreted by hypothalamus and capable of inducing the release of both LH and FSH. It has the following formula: pyroGlu - His - Trp - Ser - Tyr - Gly - Leu - Arg - Pro - Gly - NH₂.

LHRH can either stimulate pituitary gonadotropin secretion or be a potent inhibitor. When administered in a precise pulsatile pattern LHRH can restore the normal cyclic gonadotropin secretion. Pulsatile administration of LHRH using a computerized pump was used with good results in the induction of ovulation in anovulatory women with hypothalamic dysfunction. When administered chronically, LHRH or its agonists proved to be potent inhibitors of gonadotropic secretion, providing a temporary (fully reversible) gonadotropin specific medical hypophisectomy.

To date, thousands of LHRH analogs have been synthesized, that can act either as agonists or antagonists. In order to produce LHRH antagonists, which work by receptor occupancy, it is necessary to substitute several amino acids on the LHRH molecule. Antagonists also require precise topological features to achieve high binding affinity to the receptor. There are a lot of LHRH analogues recently synthesized, in which the amino acids contain aromatic or other functional groups capable of the so-called hydrotropic interaction. The use of LHRH antagonists with their immediate inhibition of gonadotrophin release may be useful in therapeutic areas, such as contraception and in treatment of hormone-dependent disorders. In the case of hormone-dependent tumours, avoiding the initial stimulatory phase produced by LHRH agonists may be a particular advantage. For a review on LHRH analogues, see Karten and Rivier, 1986.

Antide, in particular, is a potent LHRH antagonist, with formula, biological activity and preparation as described in EP Patent 377,665.

From studies carried out by the Applicant, it resulted, for example, that antide has a very poor solubility in 0.9% NaCl solution (solubility 25 µg/ml) or other isotonic media such as phosphate buffered saline (solubility was 16 µg/ml). Previous formulations of antide (e.g. antide 1 mg/ml in 5% glucose) have shown poor bioavailability and pharmacokinetic reproducibility. This is due to antide being present at the site of injection in concentrations above 25 µg/ml for example, which leads to the formation of a precipitate on contact with the physiological medium. This precipitate can be gelatinous in nature and has a detrimental effect on drug absorption, as shown by clinical investigations carried out by the Applicant.

Other gonadotrophin releasing hormone antagonists in aqueous solutions can form gel structures and in addition, the solubility is known to increase as the pH of the solution is reduced, due to increased ionisation of the molecule (Cannon J.B, et al, 1995).

It is known from the literature that, in certain cases, the addition of aromatic agents to solutions of proteins can cause a negative effect on solubility, resulting in precipitation. For example, when aromatic agents were brought into contact with recombinant human growth hormone (rhGH), conformational changes or denaturation occurred, resulting in the formation of rhGH aggregates. (Maa Y.F. and Hsu C.C., 1996). Additionally, to show that this was not a general phenomenon, it was shown that aromatic amino acid derivatives improved the solubility and enhanced the absorption of growth hormone (Leone Bay A, et al., 1996).

Nicotinamide has been reported to solubilise conventional pharmaceutical compounds (i.e. non-peptides with molecular weight less than 1000 daltons) by a process of charge transfer complexation, also called hydrotropic solubilisation. This process may result from the interaction of aromatic groups in the solubilising agent and aromatic or other suitable functional groups on the drug molecule. For example see Rasool et al., 1991.

However, the Applicant has found, and the corresponding data are here reported in the experimental section, that other molecules, containing aromatic groups such as benzoate or salicylate, which could interact by a hydrotropic mechanism (Jain N.K. and Patel V.V., 1986), show only a minor improvement in the solubilisation of an LHRH analogue (antide) in saline solution.

European Patent Application 0 649 655 describes the solubilisation of a water insoluble anti-ulcer drug using nicotinamide in order to produce a useful injectable form. Many potential derivatives of the active moiety are claimed, however, no in-vivo data were presented to demonstrate improved efficacy.

PCT Application WO 96/10417 describes the co-administration of Asp^{B28} human insulin and nicotinamide in order to achieve a rapid onset of action of the hypoglycaemic effect. The claimed nicotinamide concentration range was 0.01 to 1 M (0.1-12% w/w), but preferably from 0.05 to 0.5 M. The document gives evidence for faster absorption during an in-vivo study in pigs, however, a mechanism by which the improved absoprtion occurs is not addressed and, therefore, no generalisable teachings can be drawn from this document.

### DESCRIPTION OF THE INVENTION

It has been found that when a non-ionic aromatic hydrotropic agent is added to aqueous solutions of a peptide drug, which is normally poorly soluble in aqueous physiological saline solution, its solubility is increased and the resulting pharmaceutical compositions also show excellent stability.

In particular, it has been found that when nicotinamide is added to a saline solution (0.9% NaCl) of antide, the molecule can promote the solubility of this drug. The final concentration of antide solubilised is dependent on the concentration of nicotinamide added and increases exponentially with increased nicotinamide concentration, as shown below. The solubility of the gonadotrophin releasing hormone antagonists is known to increase as the pH of the solution is reduced, however, the data reported here in the experimental section for antide show that the increased solubility is not due to a pH effect. In addition, it has been found that nicotinamide can also increase the solubility of antide in purely aqueous environments.

Therefore, the main object of the present invention is to provide a pharmaceutical composition comprising:
a) a peptide poorly soluble in aqueous physiological saline solution, as active ingredient;
b) a non-ionic aromatic hydrotropic pharmaceutically acceptable agent; and
c) a physiological aqueous solution.

The peptide active ingredient can be an LHRH analogue. Preferably, the LHRH analogue is an antagonist. More preferably, it is antide.

A non-limiting list of suitable pharmaceutically acceptable non-ionic aromatic hydrotropic agents comprises: nicotinamide, nicotinic acid, benzoic acid, salicylic acid, gentisic acid, ascorbic acid, histidine, tryptophan, phenylalanine, tyrosine, cresol, phenol, xanthines, pyridoxine, folic acid, saccharin. Non-ionic derivatives of any of the above compounds would also be applicable for the purposes of this invention. Nicotinamide is the preferred one.

Nicotinamide is a commonly used source of vitamin B in pharmaceutical products and is administered mainly by the oral route, but also by injection. Doses of up to 500 mg daily (in divided doses) have been recommended, e.g. see Martindale.

The physiological aqueous solution can be isotonic saline or phosphate buffered saline or any suitable solution containing inorganic salts at the same tonicity as the physiological medium.

The compositions of the present invention can be suitable for any administration route, such as oral, parenteral, nasal or pulmonary administration. They can be in liquid form, as well as, in solid form as an intimate mixture (for example following spray drying, lyophilisation, etc.). They can be, for example (but not limited to), in a solid dosage form, such as a gelatin capsule for oral administration, or formulated for nasal or pulmonary inhalation. Other pharmaceutically acceptable dosage forms could be employed such as suspension, emulsion, microemulsion, micronised powder, solution, suppository, pessary, microsphere, nanosphere, implant, etc, whereby the absorption of the peptide drug is improved by the combination with the non-ionic hydrotropic agent.

Therefore, the compositions of the present invention can also be lyophilized and reconstitutable and further comprise one or more stabilizing agents, as well as one or more pharmaceutically acceptable excipients.

Exemplary composition ranges for an injectable composition are the following:

| | |
|---|---|
| antide | 0.1 - 20.0 mg |
| Nicotinamide | 10 - 300 mg |
| Aqueous phase, q.s. | 1.0 ml. |

The term 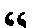 peptide 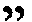 , as used in this application, means any compound made up of two or more amino acids. In it, the amino (NH₂) group of one amino acid joins the carboxyl (COOH) group of another, forming a peptide bond. Such amino acids can be naturally occurring, chemically synthesised or modified. The peptides according to the invention have generally up to 100 amino acids, preferably up to 50, more preferably up to 20.

The wording 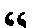 poorly soluble in aqueous physiological saline solution 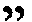 , as used in the present application, means that in such a solution, at room temperature without the addition of acids or bases, the peptide shows a solubility <1 mg/ml and/or that the solubility in aqueous physiological saline solution is one order of magnitude lower than the solubility in water alone under the same conditions.

The invention will now be described by means of the following Examples, which should not be construed as in any way limiting the present invention. The Examples will refer to the Figure specified here below.

### BRIEF DESCRIPTION OF THE DRAWING

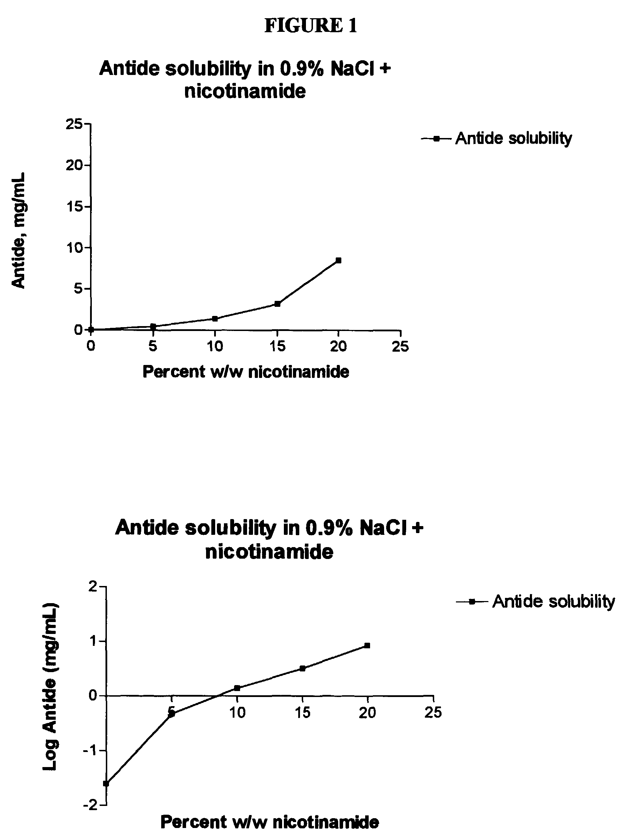

Figure 1: The antide solubility in 0.9% NaCl solution versus nicotinamide concentration is reported. The semi logarithmic plot shows that the solubility of antide bears a logarithmic relationship with the concentration of nicotinamide present. The linear nature of this profile is important, as it allows dilution effects to be considered accurately and also shows that an equilibrium solubility has been reached for the drug in these solutions.

### EXAMPLES

### Materials

Antide bulk (Bachem), batches 8901 and 9001
Dulbecco's phosphate buffered saline (Sigma, D-8537)
Histidine hydrochloride (Merck, 1.04351 - Biochemistry grade),
Nicotinamide (Fluka, 72345), USP grade,
Phenylalanine (Merck, 7256) - Biochemistry grade.
Sodium benzoate (Merck, 6290), Ph.Eur/NF grade.
Sodium salicylate (Sigma, S-3007) - General Purpose Reagent, 35H1207.
Thiamine hydrochloride (Merck 8181) - Biochemistry grade.
Rubber stoppers, butyl rubber (Pharmagummi, art. 1779 W1816 grey)
3 ml vials DIN2R glass type I (Nuova Ompi)
All the other reagents used were 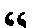 Analytical grade 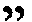 , unless otherwise specified.

### Equipment

The following equipment was used:
- Merck Hitachi HPLC system (L-6200 Pump, L-4250 Detector, AS-2000A Autosampler, Compaq PC, HPLC-Manager 2000 software)
- Waters HPLC system (626 Pump, 600S Controller, 994 Detector, 717 Autosampler, NEC PC, Maxima Baseline software)
- Free-dryer (Edwards, Lyoflex mod. 06 and mod. 04)

### Analytical method

RP-HPLC gradient elution, C-18 (e.g. Vydac 218 TP54, 250 x 4.6 mm) column. UV detection at 215 nm, injection volume 15 µl mobile phase A: pH 4.5 phosphate buffer (0.1M), mobile phase B: acetonitrile, flow rate = 1.0 ml/min, runtime = 23 min. External standard solution concentrations of 100 µg/ml were injected during analysis. The gradient was: 77% A, 23% B to 52% A, 48% B over 30 min.

### Antide stability in aqueous solutions

Preliminary stability testing of 0.1 mg/ml antide solutions at -20°C, 4°C, 25°C and 40°C was performed to evaluate their stability at pH 2, 3 and 4. The solutions at 0.1 mg/ml were prepared dissolving antide in water and adjusting the pH using 0.01 M hydrochloric acid.

### Solubilisation studies

Solubilisation studies were carried out to study the effect of:
- pH (solutions were acidified with either acetic acid or hydrochloric acid)
- hydrotropic agents (nicotinamide, sodium saccharin, sodium salicylate, sodium benzoate, histidine hydrochloride, thiamine hydrochloride, phenylalanine).

Saline used in these studies corresponds to 0.9% sodium chloride solution.

Based on the results of previous studies, excess antide was added to the test solution and observed visually to evaluate the solubility following equilibration overnight at 25°C. Following the visual analysis, a number of solutions were selected for further quantitative determination of the solubility by filtration (0.45 µm filters) and appropriate dilution using the RP-HPLC method described above.

### Manufacturing of antide freeze dried formulation

50 vials of antide/nicotinamide freeze-dried product are manufactured as follows:
1) weigh 0.7 g antide acetate (expressed as dry powder), weigh 3.5 g mannitol and add about 50 ml water for injectables (WFI)
2) bring both materials into solution by gentle stirring
3) bring to final weight of 70 g with WFI
4) fill the vials with 1 ml of solution
5) freeze-drying cycle:
   Load the product at room temperature.
   Bring the product to -40°C and maintain for 1.5 hours, then apply vacuum.
   Perform primary drying for 2 hours.
   Raise the shelf temperature to +20°C and maintain for 16 hours.
   Raise the shelf temperature to +40°C and maintain for 5 hours to complete.

### Results and discussion

### Antide stability in aqueous solutions

Three month stability testing of 0.1 mg/ml antide solutions at pH 2, 3 and 4, adjusted using diluted hydrochloric acid and stored at -20°C, +4°C, +25°C and +40°C is shown in Tables 1 & 2. The percentage of degradation increased as the pH decreased as delined by the purity values observed. It was also seen that storage at -20°C negatively affected the stability of the product when the pH was lower than 4.

### Solubilisation studies

### Effect of pH

Tables 3 to 7 summarise the observations collected in order to obtain information on the solubility behaviour of antide in water for injectables (WFI), WFI acidified by HCl or acetic acid, saline and saline acidified by acetic acid. In agreement with previous studies, the solubility of antide increased as the pH decreased.

In Table 3, the natural pH of antide acetate in solution is from 4.4 to 5.0, depending on the amount dissolved. antide could be dissolved easily in water at a concentration of 1.0 mg/ml, however, without further acidification the solubility limit was found to be 8.1 mg/ml. The pH was not measured when the solution had gelled.

When antide was added at 50 mg/ml to water adjusted to pH 3.0 with hydrochloric acid, a gel formed, indicating partial solubility. On the basis of these results, it would be necessary to add further acid in order to fully solubilise a solution at this concentration.

Tables 4 and 5 show that significant amounts of acetic acid did not effectively solubilise antide in water. The addition of 2% acetic acid in water which equates to 0.33 M solution, (deemed too concentrated for injectable purposes) was found to effectively solubilise between 10 and 20 mg/ml of antide.

The addition of 5 mg/ml antide to 1% acetic acid gave a solution of pH 3.12 in the presence of saline. The solution remained opalescent, indicating that the drug solubility was less than 5 mg/ml; the exact amount was not quantified (Table 6).

Table 7 shows that antide at a concentration of 10 mg/ml in saline is not soluble at a pH of 3.0 or 4.6, using 4% acetic acid to achieve the reduction in pH. The pH was measured when the solution was prepared, after which the gel gradually formed indicating the partial solubility of the drug. At pH 3.0 the solubility of the drug in saline solution was found to be 2.2 mg/ml, compared to the value of 0.025 mg/ml at the natural pH of 5.04 for this solution.

### Effect of hydrotropic solubilising agents on antide solubility

The effect of different hydrotropic agents (1.5% & 15% w/w) in saline solutions was also evaluated and is described in Tables 8 and 9. Nicotinamide and thiamine hydrochloride proved to be the most effective solubilising agents.

Table 8 shows the effect of low concentrations (1.5% w/w) of the hydrotropic agents selected on the pH of the solution as measured before drug addition, however, they were not effective in solubilising antide at a level of 10 mg/ml in saline.

The experiment described in Table 8 was repeated using a lower concentration of drug and a higher concentration of hydrotropic agent as described in Table 9. On quantifying the amount of antide dissolved, it was found that nicotinamide was a very good solubilising agent for antide, with 3.3mg/ml dissolved in 15% w/w nicotinamide/saline solution at pH 5.8. Thiamine hydrochloride also solubilised significant amounts of antide in saline, with 3.0 mg/ml dissolved in 15% w/w saline solution. In this case, however, the acidification caused by the acid salt of thiamine caused a substantial reduction of pH to 3.3 and the solubilising power was largely due to the acidity of this solution. Ionic hydrotropic agents did not result in good solubilisation of antide in saline.

### Nicotinamide

Further investigations were carried out to confirm the solubilising effect of nicotinamide on antide in saline solution and to establish the most effective concentration to use. The effect of the concentration of nicotinamide on antide solubility is shown in Table 10, where it was found that 20% nicotinamide in saline allows the solubilisation of 8.5 mg/ml antide.

These results are shown graphically in Figure 1. The semi logarithmic plot shows that the solubility of antide bears a logarithmic relationship with the concentration of nicotinamide present. The linear nature of this profile is important as it allows dilution effects to be considered accurately and also shows that an equilibrium solubility has been reached for the drug in these solutions.

Since nicotinamide proved to be a very good solubilising agent for antide, a further study was undertaken to verify the chemical compatibility between nicotinamide and antide. Four formulations of antide and nicotinamide at different concentration ratios and adjusted to pH 5 were prepared and tested over 3 months at +40°C, +25°C and +4°C. The stability data are reported in Table 11.

An increased viscosity with precipitate formation was observed in the formulation containing 10 mg/ml antide and 5% nicotinamide after 1 week at +40°C and +25°C; no change was observed in the samples stored at +4°C. This indicated that the solubility of antide in nicotinamide/saline solutions diminished as the temperature rose. No chemical degradation was observed in the formulations after 3 months, indicating that no chemical incompatibility existed between the two substances at the investigated ratios. Chromatographic traces of antide were identical to those of the standard, with the addition of the early eluting nicotinamide peak which appeared at a retention tune of 3.2 minutes.

The amounts of nicotinamide required to solubilise antide following injection may be greater than the isotonic concentration of nicotinamide which is 4.5%. Therefore, following injection at an extravascular site, the body fluids would re-equilibrate to adjust the osmotic balance, resuiting in a dilution of the solubilising agent. In an effort to simulate the potential dilution of an injected formulation that could occur, the effect of diluting formulations containing antide and nicotinamide in phosphate buffered solution (PBS) was studied by the following in-vitro experiments. The dilutions were made to bring the nicotinamide concentration to 5% following the addition of PBS. Solutions of antide at concentrations from 1 to 5 mg/ml were prepared in 15% nicotinamide/WFI, followed by dilution in PBS by a factor of 3. Observations were performed over a period of 3 hours, with the effect of the dilution in PBS shown in Tables 12.

The results are in good agreement with the measured solubility of antide in these systems, reported in Table 10 where a solution of 5% nicotinamide in saline can solubilise about 0.5 mg/ml of antide. The data shows that it is possible to produce a slightly supersaturated solution of antide in the body fluids by preparing, e.g. a 2 mg/ml solution of antide in 15% nicotinamide. On dilution to 5% this would contain 0.67 mg/ml of antide which remained in solution during the studies reported above.

Therefore, suitable formulations containing for example 15% nicotinamide and 2 mg/ml of antide or 5% nicotinamide and 0.5 mg/ml of antide avoid precipitation at the injection site after administration. Other suitable formulations, such as those described above, can be determined from the solubility profiles in Figure 1 and are preferred in accordance with this invention.

As a result of the Examples above, antide can be effectively solubilised by a number of agents which are non-ionic aromatic hydrotropic compounds, while the addition of ionised species reduces the aqueous solubility. antide solubility is shown to increase with decreasing pH, while the chemical stability of antide decreases as the pH is reduced below the value of pH 4.

It is also found that antide remains chemically stable in the presence of nicotinamide.

**TABLE 1**

| **Stability of 0.1 mg/ml antide solutions at pH 2, 3 and 4; Temp= +4° and -20°C** | | | | | |
|---|---|---|---|---|---|
| | | | -20°C | +4°C | |
| Formuln. | Test | T=0 | 5W | 5W | 12W |
| | | | | | |
| pH=2 | | | | | |
| | Purity (%) | 99.60 | 87.50 | 98.80 | 98.20 |
| | | | | | |
| pH=3 | | | | | |
| | Purity (%) | 99.60 | 93.50 | 99.40 | 99.10 |
| | | | | | |
| pH=4 | | | | | |
| | Purity (%) | 99.50 | 99.30 | 99.2 | 99.20 |

**TABLE 2**

| **Stability of 0.1 mg/ml antide solutions at pH 2, 3 and 4; Temp= +25° and +40°C** | | | | | | |
|---|---|---|---|---|---|---|
| | | | +25°C | | +40°C | |
| Formuln. | Test | T=0 | 5W | 12W | 5W | 12W |
| | | | | | | |
| pH=2 | | | | | | |
| | purity (%) | 99.6 | 95.8 | 92.0 | 83.4 | 65.0 |
| | | | | | | |
| pH=3 | | | | | | |
| | Purity (%) | 99.6 | 99.0 | 98.8 | 97.7 | 95.6 |
| | | | | | | |
| pH=4 | | | | | | |
| | Purity (%) | 99.5 | 99.6 | 99.4 | 99.4 | 99.1 |
| | | | | | | |

**TABLE 3**

| **antide solubility in WFI** | | | |
|---|---|---|---|
| antide (nominal amount added, mg/ml) | Appearance | pH | Solubility (mg/ml) |
| 50 | gel | ND | ND |
| 10 | opalescent | 4.40 | 8.13 |
| 1 | clear | 5.00 | 1.00 |

**TABLE 4**

| **antide solubility in WFI + 4% acetic acid** | | |
|---|---|---|
| antide (nominal amount added, mg/ml) | Appearance | pH |
| 100 | gel | ND |
| 50 | gel | ND |
| 33 | gel | ND |
| 25 | opalescent | 3.18 |

**TABLE 5**

| **antide solubility in WFI + 2% acetic acid** | | |
|---|---|---|
| antide (nominal amount added, mg/ml) | Appearance | pH |
| 40 | gel | ND |
| 20 | opalescent | 3.31 |
| 10 | clear | 3.31 |

**TABLE 6**

| **antide solubility in saline + 1% acetic acid** | | |
|---|---|---|
| antide (nominal amount added, mg/ml) | Appearance | pH |
| 10 | gel | ND |
| 5 | opalescent | 3.12 |

**TABLE 7**

| **Effect of pH on antide solubility in saline** | | | |
|---|---|---|---|
| antide (nominal amount added, mg/ml) | pH | Appearance | Solubility (mg/ml) |
| 1 | 5.04 | opalescent | 0.025 |
| 10 | 4.61 | gel | ND |
| 10 | 3.01 | opalescent | 2.23 |

**TABLE 8**

| **Effect of 1.5% hydrotropic agents on antide solubility in saline** | | | | |
|---|---|---|---|---|
| antide (nominal amount added, mg/ml) | Hydrotropic agent | pH | Appearance | Solubility (mg/ml) |
| 10 | nicotinamide | 4.89 | gel | ND |
| 10 | saccharin | 4.61 | gel | ND |
| 10 | sodium salicylate | 5.08 | gel | ND |
| 10 | thiamine hydrochloride | 3.94 | gel | ND |

**TABLE 9**

| **Effect of 15% hydrotropic agents on antide solubility in saline** | | | | |
|---|---|---|---|---|
| antide (nominal amount added, mg/ml) | Hydrotropic agent | pH | Appearance | Solubility (mg/ml) |
| 5 | nicotinamide | 5.79 | opalescent | 3.32 |
| 5 | sodium salicylate | 5.81 | opalescent | 0.21 |
| 5 | thiamine hydrochloride | 3.30 | opalescent | 3.01 |
| 5 | sodium benzoate | 6.69 | opalescent | 0.072 |
| 5 | histidine hydrochloride (5%) | 4.17 | opalescent | 0.076 |
| 5 | phenylalanine (1.7%) | 4.59 | opalescent | 0.066 |

**TABLE 10**

| **Effect of nicotinamide concentration on antide solubility in saline** | | | | |
|---|---|---|---|---|
| antide (nominal amount added, mg/ml) | Nicotinamide (%) | pH | Appearance | Solubility (mg/ml) |
| 1 | 5 | ND | opalescent | 0.47 |
| 5 | 10 | 5.68 | opalescent | 1.40 |
| 5 | 15 | 5.76 | opalescent | 3.23 |
| 10 | 20 | 5.64 | opalescent | 8.49 |

**TABLE 11**

| **Stability of nicotinamide/antide formulations at 4°, 25° and 40°C.** | | | | | | | | | | | |
|---|---|---|---|---|---|---|---|---|---|---|---|
| | | | +4°C | | | +25°C | | | +40°C | | |
| antide (mg/ml) | Nicotinamide (%) | T=0 | 1W | 4W | 12W | 1W | 4W | 12W | 1W | 4W | 12W |
| 1 | 5 | 1.0 | ND | 0.9 | 1.0 | 0.9 | 0.9 | 1.0 | 1.0 | 0.9 | 1.0 |
| 1 | 25 | 1.0 | ND | 1.0 | 1.1 | 1.0 | 1.0 | 1.1 | 1.0 | 1.0 | 1.1 |
| 10 | 5 | 9.7 | 10.3 | 10.0 | 10.5 | gel | gel | gel | gel | gel | gel |
| 10 | 25 | 9.8 | ND | 10.1 | ND | 9.8 | 9.5 | 11.1 | 10.7 | 9.4 | 10.2 |

**TABLE 12**

| **Effect of 1:3 dilution of antide/15% w/w nicotinamide solutions in PBS** | | | | | |
|---|---|---|---|---|---|
| | Ant. 5mg/ml nic. 15% | Ant. 4mg/ml nic. 15% | Ant. 3mg/ml nic. 15% | Ant. 2mg/ml nic. 15% | Ant. 1mg/ml nic. 15% |
| Time (min) | Dilution 1:3 (PBS) | Dilution 1:3 (PBS) | Dilution 1:3 (PBS) | Dilution 1:3 (PBS) | Dilution 1:3 (PBS) |
| 0 | opalescent | opalescent | clear | clear | clear |
| 5 | precipitate | precipitate | clear | clear | clear |
| 15 | precipitate | precipitate | opalescent | clear | clear |
| 30 | precipitate | precipitate | precipitate | clear | clear |
| 60 | precipitate | precipitate | precipitate | clear | clear |
| 120 | precipitate | precipitate | precipitate | clear | clear |
| 180 | precipitate | precipitate | precipitate | clear | clear |

### Legenda (for the abbreviations in all the Tables):

ND = not determined; W = weeks; Temp = temperature; WFI = water for injectables, Ant. = antide, nic. = nicotinamide

### References

Cannon I.B., "Physicochemical properties of A-75998, an antagonist of luteinising hormone releasing hormone", J. Pharm. Sci, 84, 953-958, 1995.
Maa Y.F. and Hsu C.C., "Aggregation of recombinant human growth hormone induced by phenolic compounds.", Int. J. Pharm, 140 (2) : 155-168, 1996
Jain N.K. and Patel V.V., 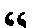 Hydrotropic solubilisation 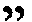 , The Eastern Pharmacist, November, 51-54, 1986 Physicians Desk Reference, Librium monograph, 47th Edition, 1993.
Sasaki H, "Effect of ophthalmic preservatives on serum concentration and local irritation of ocularly applied insulin.", Biol. Pharm. Bull. (Japan), 18/1, 169-171, 1995.
Spiegel A.J. & Noseworthy M.M., "Use of nonaqueous solvents in parenteral products", J. Pharm. Sci., 52, 917-927, 1963.
Wang Y-C.J. & Kowal R.R., "Review of excipients and pH's for parenteral products used in the United States", J. Parenter. Drug Assoc., 34, 452-462, 1980.
Golightly L., et al., "Pharmaceutical excipients: Adverse effects associated with inactive ingredients in drug products (Part 1)", Med. Toxicol., 3, 128-165, 1988.
Yalkowsky S .H. & Valvani S. C., "Precipitation of solubilised drugs due to injection or dilution", Drug Int. Clin. Pharm, 11, 417-419, 1977.
Graham C.W. et al.' "Thrombophlebitis after intravenous diazepam can it be prevented", Anaesthesia & Analgesia, 56, 409∼ 13, 1977.
Leone Bay A, et al., 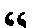 4-(4-((2-hydroxybenzoyl)amino)phenyl)butyric acid as a novel oral delivery agent for recombinant human growth hormone 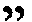 , J. Med. Chem. 39(13), 2571-2578, 1996
Rasool et al., 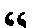 Solubility enhancement of some water-insoluble drugs in the presence of nicotinamide and related compounds 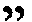 , J. Pharm. Sci, 80(4), 387 - 393, 1991.
Yalkowsky S.H. & Rubino J.T., "Solubilisation by cosolvents 1: Organic solutes in propylene glycol - water mixtures", J. Pharm. Sci, 74, 416-412, 1986.

## Claims

1. A pharmaceutical composition comprising:
a) a peptide poorly soluble in aqueous physiological saline solution, as active ingredient;
b) a non-ionic aromatic hydrotropic pharmaceutically acceptable agent; and
c) a physiological aqueous solution.

2. The pharmaceutical composition of claim 1, wherein the peptide (a) is an LHRH analogue.

3. The pharmaceutical composition according to any of the preceding claims, wherein the peptide (a) is an LHRH antagonist.

4. The pharmaceutical composition according to any of the preceding claims, wherein the peptide (a) is antide.

5. The pharmaceutical composition according to any of the preceding claims, wherein the non-ionic aromatic hydrotropic agent is selected form the group consisting of nicotinamide, nicotinic acid, benzoic acid, salicylic acid, gentisic acid, ascorbic acid, histidine, tryptophan, phenylalanine, tyrosine, cresol, phenol, xanthines, pyridoxine, folic acid, saccharin, as well as non-ionic derivatives thereof.

6. The pharmaceutical composition according to any of the preceding claims, wherein the non-ionic aromatic hydrotropic agent is nicotinamide.

7. The pharmaceutical composition according to any of the preceding claims, which is lyophilized and reconstitutable and further comprises one or more stabilizing agents.

8. The pharmaceutical composition according to any of the preceding claims, which is suitable for parenteral, oral, nasal or pulmonary administration route.

9. The pharmaceutical composition according to any of the preceding claims, further comprising one or more pharmaceutically acceptable excipients.

10. The pharmaceutical composition according to any of the preceding claims, which is suitable for injections.

11. The pharmaceutical composition of claim 10, which has the following composition ranges:
| | |
|---|---|
| Antide | 0.1 - 20.0 mg |
| Nicotinamide | 10 - 300 mg |
| Aqueous phase, q.s. | 1.0 ml. |
